# EUROPEAN PATENT APPLICATION

(11) **EP 4 755 378 A1**
(43) Date of publication of application: **10.06.2026**
(21) Application number: 24848435.4
(22) Date of filing: 26.07.2024
(51) Int. Cl.: A61K 31/00

(54) **DYRK2 INHIBITORS FOR TREATING TUMOURS WITH THE HYPERACTIVE MAPK/ERK PATHWAY**

(30) Priority: 28.07.2023 ES 202330654
(71) Applicant: Universidad de Córdoba, 14005 Córdoba (ES)
(72) Inventor: CALZADO CANALE, Marco Antonio, 14005 Córdoba (ES); SUANES COBOS, Lucía, 14005 Córdoba (ES); CORREA SÁEZ, Alejandro, 14005 Córdoba (ES); JIMÉNEZ IZQUIERDO, Rafael Manuel, 14005 Córdoba (ES); TORRES RAMOS, Miguel, 14005 Córdoba (ES)
(74) Representative: San Martin Alarcia, Esther
(86) International application number: PCT/ES2024/070481
(87) International publication number: WO 2025/027223

(57) **Abstract**

The invention relates to the use of inhibitors of the kinase protein DYRK2, alone or in combination with inhibitors of B-RAF, MEK1/2 or any other member or regulator of the MAPK/ERK1/2 pathway, to treat tumours with the hyperactive MAPK/ERK1/2 pathway.

## Description

### TECHNICAL FIELD

The invention falls within the pharmaceutical sector and is intended for use in the field of clinical oncology. Specifically, it focuses on the use of inhibitors of the MAPK/ERK 1/2 pathway in the treatment of tumours where said pathway is hyperactive, resulting in special relevance in some types of cancer such as melanoma, non-small cell lung cancer or colon cancer.

### STATE OF THE ART

Cancer constitutes one of the leading causes of morbidity and mortality worldwide, with incidence and mortality rates gradually increasing. This pathology is characterised by great heterogeneity among patients, even within the same tumour type, so targeted therapies, personalised therapies based on the altered cellular pathways in each patient, are one of the main strategies of personalised medicine to increase the survival of patients. Targeted therapies are based on identifying new treatment targets, generally proteins, responsible for driving the tumour. To that end, it is crucial to know how tumour cells work and which cellular signalling pathways are altered in these tumours.

Signalling pathways are highly coordinated by reversible phosphorylation processes involving kinases. Kinases are enzymes capable of transmitting a phosphate group to serine, threonine, or tyrosine residues of their substrates. The discovery of kinases was a turning point in the development of targeted therapies given their role in coordinating key cellular processes. Cell therapies seek to regulate key cellular processes that are deregulated in various pathologies in a specific, direct manner and with fewer adverse side effects.

Among the most important cellular signalling pathways are MAPKs (Mitogen-Activated Protein Kinases) pathways. These pathways are characterised by the sequential activation of three kinases for the regulation of vital cellular processes. In particular, different external stimuli are associated with extracellular receptors causing the activation of a MAPKKK (MAPK kinase kinase) protein through GTPases (guanosine triphosphatases) and/or other kinases close to the cell membrane. Once activated, MAPKKK phosphorylates and activates a MAPKK, which in turn phosphorylates a MAPK at a conserved TxY motif (where "T" is threonine, "Y" is tyrosine, and "x" is any amino acid). This phosphorylation causes the activation of MAPK, which will be the final effector in carrying out the phosphorylation of multiple substrates distributed throughout the cell (nucleus and cytosol) for the regulation of various key cellular processes. Given their relevance, these pathways are highly conserved evolutionarily in eukaryotes, from yeasts to mammals.

In particular, mammals have 4 MAPKs pathways, which in turn can be classified into three families based on the TxY motif of MAPK: ERK pathways (Extracellular Signal-Regulated Kinases), JNK pathway (Jun N-terminal Kinases) and p38/SAPK pathway (Stress-Activated Protein kinases).

The MAPK/JNK pathway is characterised by its involvement in key cellular processes such as inflammation, cytokine production, metabolism and regulation of programmed cell death or apoptosis. In particular, this pathway is activated by stress (ionising radiation, thermal and oxidative stress, DNA damage), inflammatory cytokines, growth factors and GPCRs (G-protein coupled receptors) agonists. Such stimuli promote the activation of Rho GTPases (Cdc42 or Rac), causing the activation of a MAPKKK (MEKK1/4, MLK2/3, ASK1, TAK1 or TpI2), which in turn activates a MAPKK (MKK4/7), and the latter finally activates MAPK (JNK1/2/3) through phosphorylation in a TPY motif. Among the key substrates of this MAPK stand out: JUN, NFAT4, STAT3, HSF1, SMAD4, p53.

On the other hand, the p38/SAPK pathway contributes to the regulation of the inflammatory process, regulation of cell cycle and differentiation, and apoptosis. It is activated by environmental stress stimuli and inflammatory cytokines, growth factors, and GPCR agonists. In this case, a MAPKKK (ML2/3, MEKKs, ASKs, TAK1 or TAO1/2) is activated, which activates a MAPKK (MKK3/6), which in turn phosphorylates and activates MAPK (p38α/β/δ/γ) in a TGY motif. Among its substrates can be highlighted: PRAK, STAT1, p53, MSK1/2 or MK2/3.

Unlike the previous families, the MAPK/ERK family can be subdivided into two classes according to the final MAPK: MAPK/ERK1/2 and MAPK/ERK5. Both pathways are characterised by the conservation of a TEY (Threonine-Glutamic-Tyrosine) activating motif in the MAPK protein. However, both pathways respond to different stimuli, have different substrates, have been described as having different cellular and embryonic functions, and have different regulatory mechanisms and activities.

Specifically, the ERK1/2 pathway is characterised by regulating a large number of vital processes and is notable for its role in cell growth, division and differentiation, survival and apoptosis **[1, 2].** This pathway is mainly activated by growth factors and mitogens, hormones, pro-inflammatory stimuli and GPCR agonists. Specifically, it consists of the binding of these ligands to cell receptors, which can be receptors of tyrosine kinase (RTKs), G protein-associated receptors or integrins. Notable receptors include the epidermal growth factor receptor (EGFR), vascular endothelial growth factor receptor (VEGFR), insulin-like growth factor receptor (IGFR), or fibroblast growth factor receptor (FGFR). In summary, the activation of this pathway begins with the binding of a ligand (growth factors, hormones, and pro-inflammatory stimuli) to a tyrosine kinase receptor on the plasma membrane, which is activated and auto-phosphorylated at tyrosine residues. These phosphorylated tyrosine residues recruit several adaptor proteins (Grb2, growth factor receptor-bound protein 2) and guanine nucleotide exchange factors (GEFs) such as SOS (Son of sevenless). GEFs mediate the transformation of inactive RAS-GDP to active Ras-GTP. Following RAS activation, serine/threonine kinase RAF is recruited to the plasma membrane and activated in a complex series of processes involving phosphorylation and dimerisation. Active RAF phosphorylates and activates MEK1/2, which in turn phosphorylates and activates ERK1/2. Active ERK1/2 can be translocated to the nucleus, activating transcription factors that induce transcription of genes involved in cellular processes, or remain in the cytoplasm where it phosphorylates a large number of substrates that regulate cellular functions **[3,4].** Ligand-receptor binding leads to the activation of GTPases (RAS, Rap), which are responsible for activating a MAPKKK (A/B/C-RAF, Mos or TIp2). For their activation, it is often necessary to form homo- or heterodimers, such as B-RAF/B-RAF or B-RAF/C-RAF. Once activated, these proteins activate a MAPKK (MEK1/2), which phosphorylates and activates MAPK (ERK1/2) in a TEY motif. MAPK activates and phosphorylates multiple substrates for the regulation of said key processes, including Jun, Fos, Myc, ER, Pax6, Mcl, Bim or Elk1. On its side, the ERK5 pathway is characterised by its activation through stress, which causes the sequential activation of a MAPKKK (MEK2/3 or Tlp2)-MAPKK (MEK5)-MAPK (ERK5). Its substrates include MEF2, STAT3, NFKB or PAK7 **[5].**

The deregulation of MAPK pathways has been related to multiple pathologies. In the case of cancer, the deregulation of the MAPK/ERK1/2 pathway stands out. This fact is based on its key role in promoting cell proliferation, division and growth, evading cell death or apoptosis, promoting tumour survival and resistance to treatments, regulating the response to stress and the relationship between the immune system and the tumour, promoting angiogenesis, degradation of the extracellular matrix and tumour cell migration, tumour invasion and metastasis, among others **[1,2].** Specifically, this pathway is hyperactive in 85% of tumours **[4].**

This hyperactivation may be caused by mutations in its canonical pathway components: RAS-RAF-MEK-ERK. The most mutated members are RAS and RAF (which includes three isoforms: A-RAF, B-RAF and C-RAF), where the genes of the K-RAS and N-RAS and B-RAF families stand out, respectively. However, alterations have also been described in molecules that indirectly modulate the pathway **[1].** In addition, RAS^{G12D}mutations are noted as being more prevalent. However, in the case of B-RAF, protein mutations in tumours have been separated into three classes, all of which promote pathway activation, but the mechanism of action is different. Specifically, classes 1, 2, and 3 differ in that they reactivate the pathway independently of RAS and the homo- or heterodimerisation (B-RAF/B-RAF or B-RAF/C-RAF), doing so through RAS-dependent mechanisms, but not dimerisation, or carrying out reactivation in a RAS and dimerisation-dependent manner, respectively. Class 1 contains the most frequent initial mutation (B-RAF^{V600E}), while class 3 is characterised by treatment resistance mechanisms and usually presents mutations in B-RAF that cause the loss of its kinase activity but favour the reactivation of the pathway by enhancing heterodimerisation and activation of C-RAF **[6].**

In particular, K-RAS is mutated in 33% of all tumours, notably pancreatic cancer (90%), colon cancer (45%), bladder cancer (50%) and non-small cell lung cancer (35%). N-RAS, on the other hand, is mainly altered in acute lymphocytic leukaemia and acute myeloid leukaemia (30%) and melanoma (15%). B-RAF is mutated in 8% of all tumours, particularly in melanoma (66%), thyroid cancer (29-83%), ovarian cancer (30%) and colorectal cancer (12%). Unlike these members, few mutations have been described in tumours of the MEK1/2 (8% melanoma; 3% colorectal cancer) and ERK1/2 (sporadic mutations) members **[7].**

Despite the high mutation rate of the pathway in various tumours, it is worth noting its role in other tumours where its members are not mutated but the pathway is hyperactive. For example, in hepatocellular carcinoma, the MAPK/ERK1/2 pathway has a low mutation rate (<5%). However, the pathway is hyperactive in approximately 50% of patients in the early stages and in almost all patients with advanced hepatocellular cancer **[3].** Another characteristic example is breast cancer, where more than half of breast tumours have a more active MAPK/ERK1/2 pathway than in adjacent benign tissue **[8].** Similarly, pancreatic cancer is characterised by constitutive activation of the pathway, not only due to mutations in K-RAS, but also due to the frequent deletion and consequent reduction of DUSP 6, which is a negative regulator of the pathway **[9].**

As a result of its importance, this signalling pathway has attracted the attention of the scientific community with the aim of developing targeted therapies that inhibit the pathway and reduce the tumour.

Initially, RAS was considered a poor therapeutic target because it had a high affinity for its substrates, specifically GTP, and a pocket that was difficult to inhibit using traditional drugs. However, covalent inhibitors against K-RAS have recently begun to be developed and are currently in clinical trials, with the exception of AMG510 (sotorasib) and MRTX849 (adagrasib), which were approved by the FDA in 2021 and 2022, respectively, for the treatment of patients with locally advanced or metastatic non-small cell lung cancer (NSCLC) who have the K-RAS^{G12C}mutation **[10].** In the case of the EMA, to date only sotorasib has been approved for the treatment of adult patients with advanced NSCLC who have the same mutation. However, these types of inhibitors are currently limited to treating patients with the G12C mutation. Unfortunately, there are still no approved inhibitors for the more common K-RAS mutations such as K-RAS^{G12D}. Various groups are trying to develop covalent inhibitors for other prevalent mutation types, with certain mutations such as G12V proving difficult to develop **[11].**

The main therapeutic targets have always been non-specific, mainly tyrosine kinase receptors, or have been aimed at inhibiting the B-RAF and/or MEK1/2 proteins. The B-RAF^{V600E}mutation is a substitution of a valine residue for a glutamic acid residue at position 600, representing the 95% of all B-RAF mutations and constituting the main target of most inhibitors developed against B-RAF. Currently, three B-RAF inhibitors have been approved by the FDA for the treatment of cancers with mutations in the V600 residue of B-RAF: Vemurafenib, Dabrafenib, and Encorafenib. Vemurafenib is a competitive ATP inhibitor of the mutant B-RAF^{V600E} and was approved by the FDA in 2011 for the treatment of patients with metastatic melanoma with the B-RAF^{V600E} mutation. Dabrafenib is another competitive ATP inhibitor of mutants B-RAF^{V600E/K/D} and was approved by the FDA in 2013 for the treatment of patients with metastatic melanoma with the B-RAF^{V600E} mutation. Encorafenib is another competitive ATP inhibitor of mutants B-RAF^{V600E/K} and was approved for the treatment of patients with metastatic melanoma with the B-RAF ^{V600E/K} mutation.

MEK1/2 has a relatively low mutation rate in human cancers, but it is an attractive therapeutic strategy because it disrupts the activation of the MAPK/ERK1/2 pathway. Currently, three MEK1/2 inhibitors have been approved by the FDA and EMA: trametinib, binimetinib, and cobimetinib. Trametinib is a non-competitive allosteric ATP inhibitor of MEK1/2 and has been approved by the FDA and EMA in combination with Dabrafenib for the treatment or adjuvant therapy of unresectable or metastatic melanoma with the B-RAF^{V600E/K} mutation and for metastatic NSCLC with the B-RAF^{V600E} mutation. In addition, the FDA has also approved the combination of Trametinib with Dabrafenib for the treatment of locally advanced or metastatic anaplastic thyroid cancer with a B-RAF mutation and no response to locoregional treatments. Binimetinib and Cobimetinib are two allosteric inhibitors of MEK1/2 approved in combination with Encorafenib or Vemurafenib, respectively by the FDA for any unresectable tumour or metastatic solid tumours with the mutation in this protein and by the EMA for the treatment of unresectable melanomas and advanced non-small cell lung tumours with mutations in the B-RAF protein. As with B-RAF inhibitors, MEK1/2 inhibitors also have limited efficacy due to the development of resistance and dose-limiting toxicities. In addition, these inhibitors have been found to be ineffective when the patient has progressed after the use of B-RAF inhibitors, making it necessary to administer them in combination or beforehand [7].

The combination of B-RAF and MEK1/2 inhibitors is currently the best therapeutic approach. In fact, all combinations of inhibitors offer better clinical results and greater efficacy than those obtained with inhibitors as individual agents. However, although with a delay in their onset, episodes of resistance have been observed in patients treated with these combinations of inhibitors **[12].**

ERK1/2, the final effectors of the MAPK/ERK1/2 pathway, are also targets of great interest for the development of inhibitors targeting the pathway. The development of ERK1/2 inhibitors is relatively limited and, to date, ONC201/TIC10 (Imipridone) is the only ERK1/2 inhibitor that has been approved by the FDA for the treatment of glioma with the H3K27M ² mutation.

Most of these therapies, including the new covalent RAS inhibitors, show potent initial action on tumours. However, over time, patients relapse as a result of tumour recurrence after reactivating the pathway through resistance mechanisms. For example, patients with melanomas and the B-RAF^{V600E} mutation show a response to the combination of B-RAF and MEK1/2 inhibitors of more than 70%. However, this response is limited in duration, with more than half of patients relapsing after one year of treatment **[7].**

Recently, it has been reported that certain clones in a tumour of resistant tumour cells become sensitive to treatment again after a period of time without exposure. This allows us to talk about initial persistence mechanisms and resistance mechanisms. Persistence mechanisms are an initial response by the tumour to try to reactivate the pathway without this being an irreversible mechanism. For example, epitranscriptomic mechanisms that upregulate or downregulate certain genes for cell growth and/or pathway reactivation. Resistance mechanisms, on the other hand, are generally a later response to drug exposure, which are irreversible, usually due to gene mutations. There are various mechanisms of resistance and persistence. Among them, mutation rates are usually low, between 20-30% of them, and are generally new mutations in the NRAS, MEK1/2, EGFR, and B-RAF members. The most common form of pathway reactivation is usually an increase in positive regulators of the pathway (e.g. COT, RAS-GEF) or a decrease in negative regulators of the pathway (e.g. DUSP6, DUSP1, NF1, RAS-GAP, Sprouty, RKIP) with the aim of its application. Other resistance mechanisms include gene amplification, notably the increase in cell receptors (e.g. MET, HER, RTK, EGFR), promotion of C-RAF/B-RAF heterodimerisation, or alternative splicing variants (especially of B-RAF). Resistance mechanisms associated with non-coding RNA have also been described, such as microRNAs (e.g. downregulation of miR4510 or miR30a and upregulation of miR3305p or miR487), BANCR (BRAF-activated non-coding RNA), IncRNAs (e.g. upregulation of IGF2AS, LL22NCO3-NI4H11.1, URHC or downregulation of RUNXi-IT1 or CAS2) [3].

Another important aspect to consider in current drugs designed to inhibit the pathway, especially B-RAF inhibitors, is their paradoxical effect. This effect is characterised by the drug promoting the activation or inhibition of the pathway depending on whether the pathway is activated by upstream alterations of B-RAF (e.g. EGFR receptor amplification or RAS mutation) or whether it is due to a mutation in B-RAF, respectively. This effect is believed to be due to the fact that the B-RAF inhibitor favours an inactive B-RAF conformation that promotes greater association with C-RAF. Thus, when the pathway is hyperactive upstream of B-RAF, greater activation of C-RAF is allowed and, as a consequence, activation of the pathway and tumour growth **[13].** This paradoxical activation is characteristic of second-generation inhibitors, such as Vemurafenib or Dabrafenib, which are indicated for the treatment of tumours with altered B-RAF. Currently, third-generation inhibitors or "*paradox breakers*," characterised by not producing the paradoxical effect, such as PLX-7904 and PLX-8394, are in the preclinical phase **[14].**

Despite current knowledge about the members and possible mechanisms of resistance, these are often insufficient to explain the reactivation of the pathway. This fact highlights the current lack of knowledge about it and the need to study new regulators of it that can serve as therapeutic targets. As described, there are several inhibitors of components of the MAPK/ERK1/2 pathway that can be administered alone or in combination. However, there are still numerous limitations, mainly due to the reactivation of the pathway by resistance mechanisms, which highlight the need to identify new treatment targets.

### DESCRIPTION OF THE INVENTION

The object of the invention is the use of DYRK2 protein kinase inhibitors, alone or in combination with B-RAF, MEK1/2 or any other member or regulator of the MAPK/ERK1/2 pathway inhibitors, for the treatment of tumours with an altered MAPK/ERK1/2 pathway.

The present invention identifies the protein kinase DYRK2 as a new and unknown positive regulator of the MAPK/ERK1/2 pathway.

DYRK2 (Dual specificity tyrosine-phosphorylation-regulated kinase 2) is a serine/threonine kinase belonging to the CMGC kinase group and more specifically to the DYRK subfamily. Like the other members of the DYRK subfamily, DYRK2 directly phosphorylates its substrates. In particular, DYRK2 prefers to phosphorylate serine/threonine residues with the consensus sequence "Rx(x)S/TP", although this pattern is not necessarily present at all phosphorylation sites [**15**]. Among its phosphorylation activities, DYRK2 acts as a priming kinase for GSK3β. In addition to its kinase activity, DYRK2 also acts as a scaffolding protein forming part of the E3 ubiquitin ligase complex EDVP **[16].** Through the phosphorylation of its substrates or by acting as a priming kinase, DYRK2 is implicated in several human cancers, regulating multiple key cellular processes in carcinogenesis **[17].** In this regard, DYRK2 has been described as regulating substrates related to cell proliferation, such as c-Jun or c-Myc, apoptosis, such as p53 or MOAP1, epithelial-mesenchymal transition, cell migration or metastasis, such as SNAIL, or the formation of cancer stem cells, such as KLF4. The relevance of these substrates highlights the importance of DYRK2 in controlling the development and progression of human cancers. However, to date, this kinase has not been linked to MAPK pathways.

DYRK2 kinase increases in response to classic inhibitors of the pathway, suggesting a new possible mechanism of resistance. For its part, the inhibition of DYRK2 in combination with classic inhibitors of the MAPK/ERK1/2 pathway shows a more potent anti-tumour response than classic treatments in various tumour lines with the MAPK/ERK1/2 pathway hyperactive. This is mainly because most currently approved inhibitors are not specific or are intended to inhibit a single protein in the pathway. However, by inhibiting DYRK2, as tested in the present invention, different members of the MAPK/ERK1/2 pathway are negatively regulated. This could explain, and in turn solve, a new and unknown mechanism of resistance, which would curb the problem of patient recurrence.

Specifically, it describes the regulation of two different members of the MAPK/ERK1/2 pathway by DYRK2, both of which promote hyperactivation of the pathway. Firstly, it has been observed that DYRK2 is capable of stabilising all members of the RAF family, as well as mutant versions of B-RAF that are common in tumours. Conversely, the absence or reduction of DYRK2 levels drastically decreases B-RAF levels. Here, it is described that DYRK2 is capable of co-localising *in vivo* with B-RAF, as well as phosphorylating B-RAF *in vitro* at residues S147, S465 and S729. Residue S729 has been described as a key residue in the stabilisation and activation of B-RAF in cells. Specifically, B-RAF is initially in an inactive state, and phosphorylation at S729 allows the binding of the 14-3-3 chaperone protein and the subsequent formation of active homo- and heterodimers that interact with MEK1/2, promoting the activation of ERK1/2 **[18].** To date, this phosphorylation has only been reported to occur via AMPK kinase. However, experimental results show that other kinases are involved in this activation process. DYRK2 would be the kinase responsible for this process of stabilisation and activation of B-RAF. Unlike residue S729, no information is available on the phosphorylation of residue S147 of B-RAF, and the information on residue S465 is contradictory. Residue S465 has been described as having a relatively disordered structure, which allows for protein flexibility, and is expected to be located near the interface with MEK1. This may suggest the involvement of the residue in the binding of B-RAF to MEK1 **[19].** A phospho-proteomic study has demonstrated an increase in the S465 residue of B-RAF following ERK1/2 inhibition as a feedback response mechanism for possible reactivation **[20].** Conversely, it has been reported that B-RAF auto-phosphorylates at the P-loop residues S465 and S467 in the absence of its substrate MEK1 in an inhibitory manner. However, no alterations in downstream signalling are observed if B-RAF is mutated at V600E **[21,22].** This report demonstrates that DYRK2 can phosphorylate these residues *in vitro* and that DYRK2 promotes the activation of the MAPK/ERK pathway, as observed in the increase in p-ERK phosphorylation.

In parallel, this study describes the regulation of MEK1 by the DYRK2 kinase independently of the regulation of B-RAF by the same kinase. Specifically, we demonstrate that DYRK2 is capable of phosphorylating MEK1 *in vitro* and *in vivo* at residues T292 and S298. Although both phospho-sites had initially been described as opposites, T292 was considered inhibitory and S298 activating **[23].** It has been observed that the combination and coordination of phosphorylation at both residues is necessary for the activation of ERK2 by MEK1 and the consequent transfer of ERK2 to the nucleus to promote gene alteration related to proliferation and survival. More specifically, it has been described that PAK1 can phosphorylate MEK1 at S298, favouring the interaction of MEK1/ERK2. Once ERK2 is activated by its phosphorylation in the activation loop, ERK2 is able to phosphorylate MEK1 at T292 to decrease the affinity of the MEK1/ERK2 interaction, thus leaving ERK2 activated and free for transport to the nucleus **[24].** In this sense, DYRK2 is a new regulator of the MEK1/ERK2 interaction and the consequent activation of ERK2 through its phosphorylation at residues T292/S298.

It also shows how tumour cell lines respond to prolonged inhibition of the pathway by classic inhibitors targeting B-RAF or MEK1, such as Dabrafenib or U0126, respectively, with an increase in DYRK2 levels. This observation could be related to the increase in B-RAF phosphorylation at S465 following ERK inhibition **[20].**

Thus, DYRK2 is a therapeutic target for tumours where the MAPK/ERK1/2 pathway is hyperactive and, based on these results, the inhibition of DYRK2 offers significant advantages over other previously described inhibitors.

On the one hand, current inhibitors are either non-specific against a multitude of targets, which is associated with a greater number of side effects, or targeted against a single member of the MAPK/ERK1/2 pathway, leading to frequent resistance mechanisms and requiring the combination of more than one inhibitor.

DYRK2 activates the pathway through direct regulation of both B-RAF and MEK1, which means that a single drug can be used to decrease the activation of the pathway through two main axes. In fact, results show that in the presence of the B-RAF inhibitor Dabrafenib, DYRK2 is capable of increasing the phosphorylation and activation status of ERK1/2. Therefore, the use of B-RAF inhibitors is not sufficient to completely prevent the positive regulation of the pathway mediated by DYRK2.

On the other hand, one of the resistance mechanisms for overcoming B-RAF inhibitors is the promotion of B-RAF/C-RAF heterodimers. Specifically, C-RAF has little basal activity as a monomer, but when it binds to B-RAF, this activity is enhanced (paradoxical effect). B-RAF inhibitors or direct mutations in B-RAF characterised by a decrease in its activity (class 3 mutations) promote heterodimerisation and activation of the pathway. Given that DYRK2 positively modulates the stability of all members of the RAF family, as well as the hyperactive or inactivating isoforms of B-RAF, this effect indicates that the inhibition of DYRK2 hinders this resistance mechanism by destabilising not only B-RAF but also C-RAF. In this way, it behaves as a third-generation indirect RAF inhibitor.

Finally, it is also important to note that resistance mechanisms are often promoted by regulators, which are sometimes unknown. This study has described that DYRK2 increases in response to classic inhibitors of the pathway, pointing to DYRK2 as a previously unknown resistance mechanism.

Therefore, **a first aspect** of the invention describes a DYRK2 inhibitor for use as an inhibitor of the MAPK/ERK1/2 pathway. Preferably, the DYRK2 inhibitor is selected from any of those available to date, including YK-2-69, C17 **[25],** compound C43 **[26],** LDN192960, GSK626616, Leucettine L41, EHT 5372, EHT 1610, TG003, INDY, DYR219, SM07883, RD0392, Abbott COT/TPL2 compound 41, Fluoro-DANDY analogue 5 g, SC97202, LY2835219, Milciclib, PAC1, Compound 6i, Curcumin, Harmine, Ro3306, BX-795, A-443654, AnnH75, WNK-463, GNF2133 **[17]** and their derivatives. More preferably, the DYRK2 inhibitor used is LDN192960.

A **second aspect** of the invention is the use of the aforementioned DYRK2 inhibitor for use as a medicament in the treatment of cancers characterised by hyperactivation of the MAPK/ERK1/2 pathway, preferably pancreatic cancer, colon cancer, bladder cancer, non-small cell lung cancer, acute lymphocytic leukaemia, acute myeloid leukaemia, melanoma, thyroid cancer, ovarian cancer, hepatocellular carcinoma, breast cancer or pancreatic cancer.

A **third aspect** of the invention describes a pharmaceutical composition, hereinafter referred to as the "composition of the invention", comprising at least one DYRK2 inhibitor from among those mentioned above for use in the treatment of cancers characterised by the hyperactivation of the MAPK/ERK1/2 pathway. In a preferred embodiment of the composition of the invention, the pharmaceutical composition further comprises at least one other inhibitor of a receptor of the MAPK/ERK1/2 pathway other than the DYRK2 inhibitor.

In a more preferred embodiment, the inhibitor of a receptor of the MAPK/ERK1/2 pathway is a B-RAF inhibitor, preferably Vemurafenib, Dabrafenib, Encorafenib, PLX-4720, RAF-265 (CHIR-265), GDC-0879, AZ-628, SB590885, ZM336372, GW5074, TAK-632, Donafenib, Agerafenib (RXDX-105), Belvarafenib (HM95573), GNE-9815 or derivatives thereof.

In another more preferred embodiment, the inhibitor of a receptor of the MAPK/ERK1/2 pathway is an inhibitor of MEK1/2, preferably Trametinib, Binimetinib, Cobimetinib, U0126, Avutometinib, Selumetinib (AZD6244), Mirdametinib (PD0325901), PD98059, PD184352 (CI-1040), Pimasertib (AS-703026), BIX 02189, Pelitinib (EKB-569), TAK-733, AZD8330, Refametinib (RDEA119), Zapnometinib (PD0184264), GDC-0623, BI-847325 or their derivatives.

In another even more preferred embodiment, the pharmaceutical composition comprises, in addition to the DYRK2 inhibitor, at least one B-RAF inhibitor and one MEK1/2 inhibitor, preferably Dabrafenib as the B-RAF inhibitor and U0126 as the MEK1/2 inhibitor.

In another more preferred embodiment, the inhibitor of a receptor of the MAPK/ERK1/2 pathway is a RAS inhibitor, preferably AMG510 (Sotorasib), MRTX849 (Adagrasib), MCP110, Lonafarnib (SCH66336), JDQ443, Pan-RAS-IN-1, ASP2453, BI-3406, BI-2852, Kobe2602, BAY-293, KRpep-2d, ARS-853, ARS-1620, TH-Z816, or derivatives thereof.

In another more preferred embodiment, the inhibitor of a receptor of the MAPK/ERK1/2 pathway is an inhibitor of ERK1/2, preferably ONC201/TIC10 (Imipridone), SCH772984, ASN007, MRTX-1257. AZD0364 (ATG-017), MK-8353 (SCH900353), Temuterkib (LY3214996), Pluripotin (SC1), VX-11e, Ulixertinib (BVD-523), Ravoxertinib (GDC-0994), FR 180204 or their derivatives.

In another, even more preferred embodiment, the composition of the invention further comprises inhibitors of positive regulators of the MAPK/ERK1/2 pathway such as COT, RAS-GEF, AMPK, PKA, PAK1 or CAMPK and/or stabilisers of negative regulators of the MAPK/ERK1/2 pathway such as DUSP6, DUSP1, NF1, RAS-GAP, KSR, Sprouty or RKIP.

As used herein, the term "pharmaceutical composition" refers to any substance used for the diagnosis, prevention, alleviation, treatment or cure of a disease in a human or animal. The pharmaceutical composition of the invention may be used alone or in combination with other pharmaceutical compositions.

In a particular embodiment, the pharmaceutical composition of the invention further comprises a pharmaceutically acceptable carrier or excipient. The term "pharmaceutically acceptable excipient" refers to a substance that aids in the absorption of the pharmaceutical composition comprising the composition of the invention, stabilises said pharmaceutical composition, or aids in the manufacture thereof in the sense of giving it consistency, shape, flavour, or any other specific functional characteristic. Thus, excipients could have the function of holding the ingredients together, such as for example, starches, sugars or celluloses, a sweetening function, a colouring function, a protective function, such as isolating it from air and/or moisture, a filling function for a tablet, capsule or any other form of formulation, such as dibasic calcium phosphate, a disintegrating function to facilitate the dissolution of the components and their absorption, without excluding other types of excipients not mentioned in this paragraph.

A "pharmaceutically acceptable carrier" (or "pharmacologically acceptable carrier") refers to any substance or combination of substances, known in the pharmaceutical sector, used in the manufacture of pharmaceutical dosage forms and includes, among others, solids, liquids, solvents or surfactants. The carrier may be an inert substance or have an action similar to any of the compounds of the present invention, having the function of facilitating the incorporation of the drug as well as other compounds, allowing for better dosing and administration, or providing consistency and shape to the pharmaceutical composition. When the pharmaceutical form is liquid, the carrier is the diluent. The term "pharmacologically acceptable" refers to the fact that the compound to which it refers is permitted and evaluated as not causing harm to the organisms to which it is administered.

The pharmaceutical composition of the invention can be administered through any route of administration, and as such, said composition will be formulated in the pharmaceutical form appropriate to the chosen route of administration. Thus, the pharmaceutical composition of the invention can be administered orally, nasally, ocularly, topically, intradermally, intracranially, intravenously, or intraperitoneally.

The pharmaceutical composition may further comprise another compound useful in the treatment of cancer. The pharmaceutical composition may include a single composition or separate compositions.

The pharmaceutical composition may include an effective amount of the DYRK2 inhibitor, alone or in combination with another inhibitor of a receptor of the MAPK/ERK1/2 pathway. The term "effective amount" as used herein refers to an amount sufficient to inhibit DYRK2 and the MAPK/ERK1/2 pathway and to prevent or treat cancer in an individual in need of such prevention or treatment. The effective amount may be appropriately selected based on a cell or individual selected by a person skilled in the art. For example, the effective amount may be determined based on the severity of the disease, the age of the patient, body weight, health conditions, sex, the patient's sensitivity to the drug, the duration of administration, the route of administration, excretion rate, duration of treatment, and other factors, including the use of a drug in combination with or at the same time as the pharmaceutical composition, and other factors known in the field of medicine.

The term "prevention," as used herein, refers to the ability of the pharmaceutical composition of the invention to prevent, minimise, or hinder the progression of cancer. Therapy is considered "personalised" when the compound administered to an individual to treat a disease (cancer) is specially tailored to the genotypic and phenotypic characteristics of the individual to be treated, thus avoiding wasted time with ineffective therapies. In the present invention, the characteristic that determines the therapy to be administered to the individual is the hyperactivation of the MAPK/ERK1/2 pathway in a cancer cell.

The term "patient" or "subject," as used herein, refers to any animal, preferably a mammal, and includes, but is not limited to, domestic and farm animals, primates, and humans, for example, humans, non-human primates, cows, horses, pigs, sheep, goats, dogs, cats, or rodents such as rats and mice. In a preferred embodiment, the subject is a human being of any age or race. In a particular embodiment, the subject suffers from cancer characterised by hyperactivation of the MAPK/ERK1/2 pathway compared to a reference value.

The term "cancer," as used herein, refers to a disease characterised by uncontrolled cell division (or by increased resistance to survival or apoptosis) and by the ability of such cells to invade other neighbouring tissues (invasion) and spread to other areas of the body where the cells are not normally found (metastasis) through the lymphatic and blood vessels, circulate through the bloodstream, and then invade normal tissues in other parts of the body. Depending on whether or not they can spread by invasion and metastasis, tumours are classified as benign or malignant: benign tumours are tumours that cannot spread by invasion or metastasis, i.e. they only grow locally; malignant tumours are tumours capable of spreading by invasion and metastasis. Biological processes known to be related to cancer include angiogenesis, immune cell infiltration, cell migration, and metastasis. As used herein, the term cancer includes, but is not limited to, the following types of cancer: pancreatic cancer, colon cancer, bladder cancer, non-small cell lung cancer, acute lymphocytic leukaemia, acute myeloid leukaemia, melanoma, thyroid cancer, ovarian cancer, hepatocellular carcinoma, breast cancer, and pancreatic cancer.

Unless otherwise defined, all technical and scientific terms used herein have the same meaning commonly understood by a person skilled in the art to which this invention pertains. Methods and materials similar or equivalent to those described herein may be used in the practice of the present invention. Throughout the description and claims, the word "comprising" and its variations are not intended to exclude other technical features, additives, components, or steps. Other objects, advantages and features of the invention will be apparent to those skilled in the art upon examination of the description or may be learned through practice of the invention. The following examples, drawings and sequence listing are provided for illustration and are not intended to be limiting of the present invention.

### DESCRIPTION OF THE FIGURES

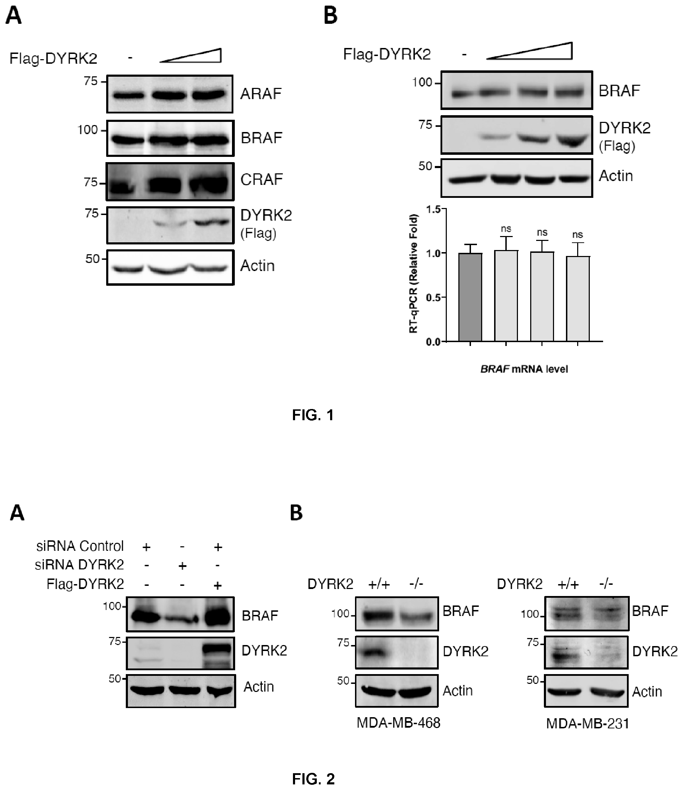

**Figure 1****. Post-transcriptional regulation of RAF mediated by DYRK2.** (A) Extracts from HEK-293T cells transfected with increasing doses of Flag-DYRK2 were analysed by Western-blot using the indicated antibodies. (B) HEK-293T cells were transfected with the indicated plasmids and their cell lysates were used in parallel to measure B-RAF protein and mRNA levels by Western-blot and RT-qPCR, respectively. Mean ± SD (n=3) is shown. Student's t tests were performed (ns = not significant). The results in both figures are representative images from 3 independent experiments.
**Figure 2****. DYRK2 basally regulates B-RAF levels.** (A) DYRK2 expression or silencing was performed using a specific siRNA. After 48 hours, its impact on B-RAF was determined via Western-blot. (B) B-RAF and DYRK2 levels were analysed by Western-blot in DYRK2 WT (+/+) or DYRK2 knock-out (-/-) cells. The results shown are a representative image of at least 3 different replicates.

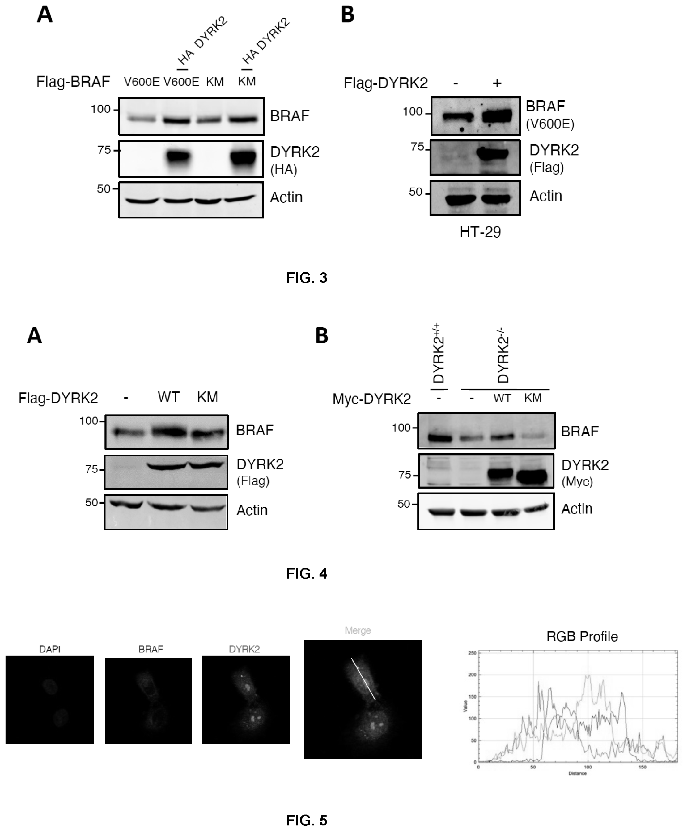

**Figure 3****. Stabilisation of the most common mutant versions of B-RAF by DYRK2.** (A) HEK-293T cells were transfected with the characteristic mutant versions of B-RAF (Flag-BRAF V600E/Flag-BRAF KM) alone or in combination with HA-DYRK2. Their cell extracts were analysed with the indicated antibodies by Western-blot. (B) Flag-DYRK2 was expressed in HT-29 cells and Western-blot was used to determine the stabilisation of endogenous B-RAF-V600E. Representative results from different biological replicates are shown.
**Figure 4****. Involvement of DYRK2 kinase activity in B-RAF modulation.** (A) HEK-293T cells were transfected with a wild-type version of DYRK2 (Flag-DYRK2 WT) or a mutant version lacking kinase activity (Flag-DYRK2 KM). The indicated proteins were analysed by Western-blot. (B) The protein level of B-RAF was compared between HeLa DYRK2 WT (DYRK2 +/+) or DYRK2 KO (DYRK2-/-) cells transfected or not with Myc-DYRK2 with (WT) or without (KD) kinase activity. The experiments shown are a representation of several biological techniques (n>3).
**Figure 5****. DYRK2 and B-RAF co-localise in the cytoplasm.** The subcellular localisation of B-RAF and DYRK2 was analysed by indirect immunofluorescence using confocal microscopy. DNA staining with DAPI was used as a nuclear marker. RGB Profile shows the intensity of the various fluorescence signals throughout the image.

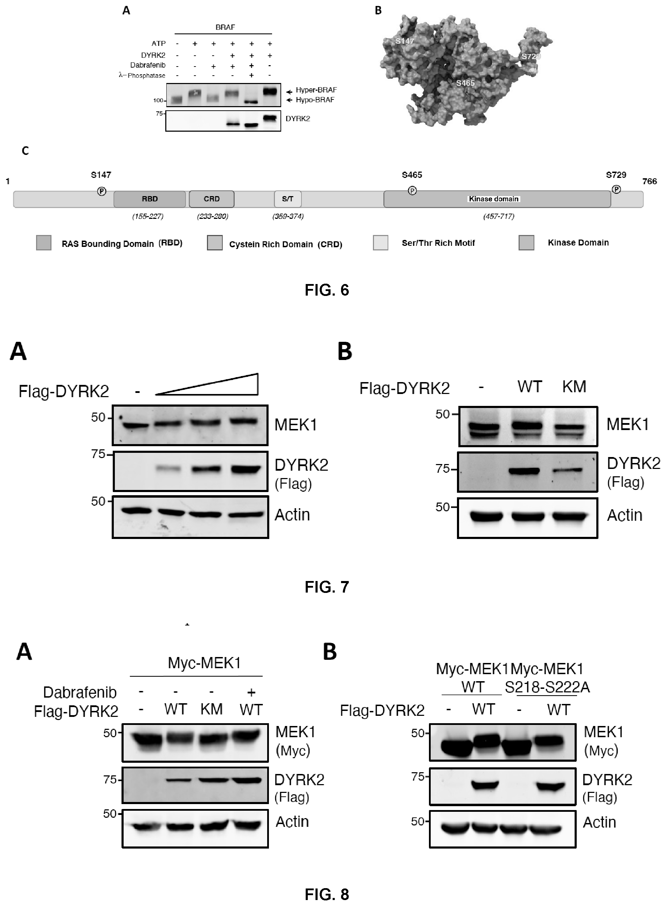

**Figure 6****. DYRK2 phosphorylates B-RAF** ***in vitro.** (A) In vitro* kinase assay where the complete recombinant B-RAF protein was exposed to different conditions with ATP (10 µM), recombinant DYRK2 protein, Dabrafenib (10 uM) and/or λ-phosphatase. The reactions were incubated for 1 hour at 30°C and alterations in the electrophoretic migration of B-RAF were analysed by Western-blot. (B) and (C) Three-dimensional and two-dimensional model of the B-RAF protein, where the residues identified by mass spectrometry are marked in yellow and the different domains of the protein are colour-coded as indicated.
**Figure 7****. DYRK2 kinase activity promotes delayed MEK1 migration.** (A) HEK-293T cells were transfected with increasing doses of Flag-DYRK2. MEK1 was analysed under these conditions via Western-blot. (B) A wild-type version of DYRK2 (Flag-DYRK2 WT) or a mutant version lacking kinase activity (Flag-DYRK2 KM) was expressed, and the proteins were analysed with the indicated antibodies by Western-blot. The results shown are a meaningful representation of more than three biological replicates.
**Figure 8****. Regulation of MEK1 by DYRK2 is independent of the DYRK2-B-RAF axis.** (A) Myc-MEK1 was expressed alone or in combination with DYRK2 with (WT) or without (KM) kinase activity in HEK-293T cells. After 12 hours of exposure to Dabrafenib (10 µM), the cells were lysed and the indicated proteins were analysed by Western-blot. (B) HEK-293T cells were transfected with a wild-type version of MEK1 (Myc-MEK1 WT) or a mutant version in its activation loop (Myc-MEK1 S218A-S222A) with or without Flag-DYRK2. Changes in the electrophoretic mobility of MEK1 under the various conditions were studied by Western-blot. Representative figures from at least three separate biological replicates are shown.

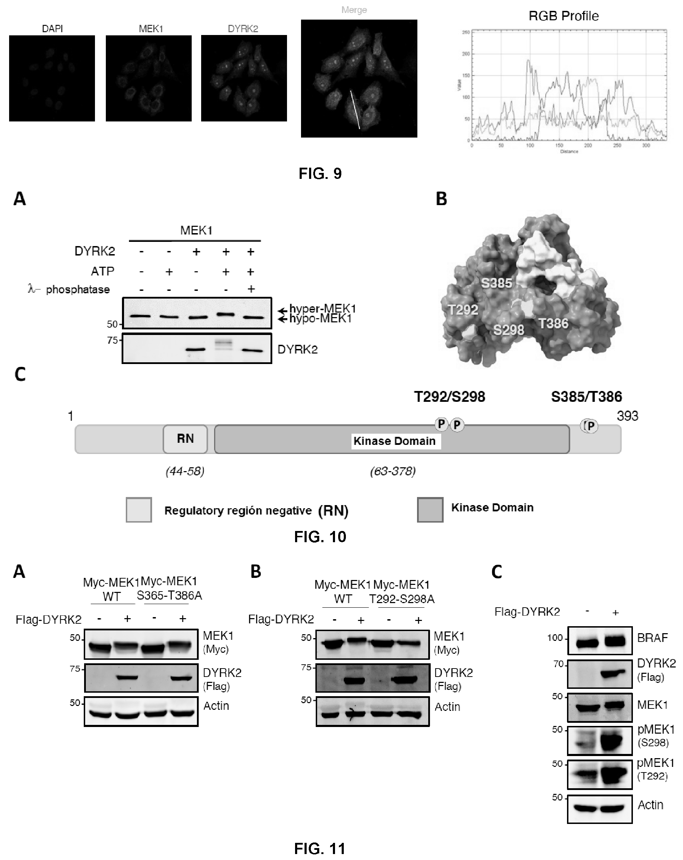

**Figure 9****. MEK1 and DYRK2 co-localise in the cytoplasm.** Indirect immunofluorescence was performed in CHO cells to determine the subcellular localisation of MEK1 and DYRK2 using confocal microscopy. Merge and RGB profile are different approaches to observe the overlap of the signals of both proteins.
**Figure 10****. DYRK2 phosphorylates MEK1 *in vitro.*** (A) Active and inactive recombinant proteins of DYRK2 and MEK1, respectively, were incubated in the presence or absence of ATP (10 µM) and λ- phosphatase. Changes in the electrophoretic migration of both proteins were analysed by Western-blot. (B) Three-dimensional and (C) two-dimensional localisation of residues identified by MS/MS (yellow) in the MEK1 protein. The domains and characteristic regions of MEK1 are coloured in both models based on the colour code indicated.
**Figure 11****. Identification of DYRK2-mediated phospho-residues in MEK1 *in vivo.*** The wild-type version of MEK1 (Myc-MEK1 WT) and a mutant version (Myc-MEK1 S385A/T386A (A) or Myc-MEK1 T292A/S298A (B)) were expressed in the presence or absence of Flag-DYRK2 in HEK293-T cells. The electrophoretic migration patterns of MEK1 under the various conditions were compared by Western-blot. (C) The phosphorylation levels of various MEK1 residues (pMEK1 T292 and pMEK1 S298) were analysed with or without Flag-DYRK2 expression. Representative panels from more than three independent experiments are shown.

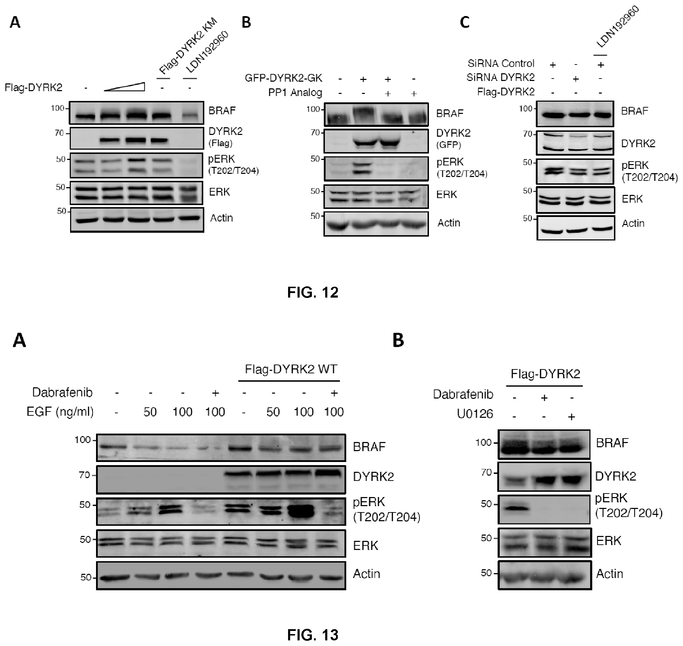

**Figure 12****. DYRK2 activates the MAPK/ERK pathway.** (A) HEK-293T cells were transfected or not with a gradient of the wild-type version of Flag-DYRK2 or a mutant version lacking kinase activity (Flag-DYRK2 KM). Two hours before lysing and obtaining the cell extracts, the cells were exposed to the DYRK2 inhibitor LDN192960 (10 µM). The proteins and their indicated phospho-residues were analysed by Western-blot. (B) A mutant version of DYRK2 sensitive to the PP1 analogue (GFP-DYRK2-GK) was expressed or not in HEK-293T cells. The cells were lysed after 4 hours of exposure to the PP1 analogue (3 µM) and their extracts were analysed by Western-blot. (C) DYRK2 was silenced or inhibited by using specific siRNA (SiRNA DYRK2) or by using the inhibitor LDN192960 (10 µM) for 2 hours, respectively, in HEK-293T cells. The protein levels of the indicated proteins were analysed by Western-blot. Representative experiments from at least three separate biological replicates are shown.
**Figure 13****. Dual regulation of the MAPK/ERK-DYRK2 axis.** (A) HEK-293T cells that had been 48 hours previously transfected with Flag-DYRK2 or not were exposed to Dabrafenib (10 µM) and/or increasing doses (50-100 ng/ml) of epidermal growth factor (EGF) for 24 hours or 5 minutes, respectively. The indicated proteins were analysed with their specific antibodies via Western-blot. (B) HEK-293T cells were transfected with Flag-DYRK2 and 24 hours later, they were stimulated for another 24 hours with the inhibitors Dabrafenib (10 µM) or U0126 (10 µM). The protein extracts were analysed by Western-blot. Representative figures from more than three independent experiments are shown.

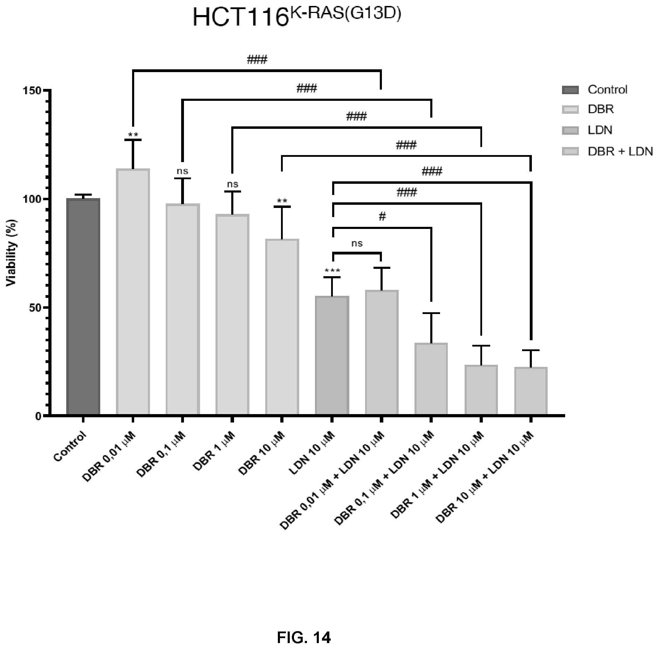

**Figure 14****. Viability of HCT116 cells in response to B-RAF inhibition, DYRK2 inhibition, or a combination of both.** HCT116 cells were stimulated with different doses of Dabrafenib (DBR), in the presence or absence of LDN192960 (10 µM) (LDN) for 48 hours. Viability was analysed using the XTT assay. The mean ± SD of three independent experiments is shown. Statistically significant differences are indicated as *, p<0.05; **, p<0.01; ***, p<0.001 for DBR *versus* DBR + LDN; #, p<0.05; ##, p<0.01; ###, p<0.001 for LDN *versus* DBR + LDN; ns, no significant differences. DBR = Dabrafenib; LDN = LDN192960.

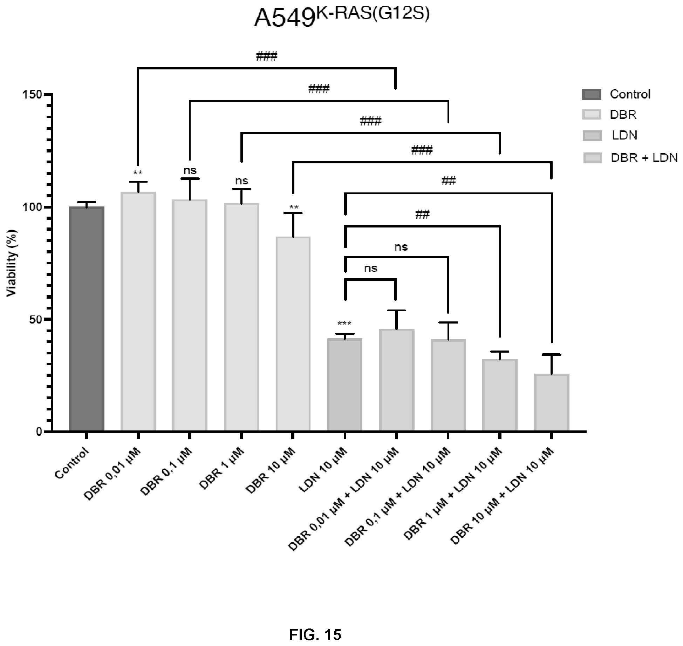

**Figure 15****. Viability of A549 cells in response to B-RAF inhibition, DYRK2 inhibition, or their combination.** A549 cells were stimulated with different doses of Dabrafenib (DBR), in the presence or absence of LDN192960 (10 µM) (LDN) for 48 hours. Viability was analysed using the XTT assay. The mean ± SD of three independent experiments is shown. Statistically significant differences are indicated as *, p<0.05; **, p<0.01; ***, p<0.001 for DBR *versus* DBR + LDN; #, p<0.05; ##, p<0.01; ###, p<0.001 for LDN *versus* DBR + LDN; ns, no significant differences. DBR = Dabrafenib; LDN = LDN192960.

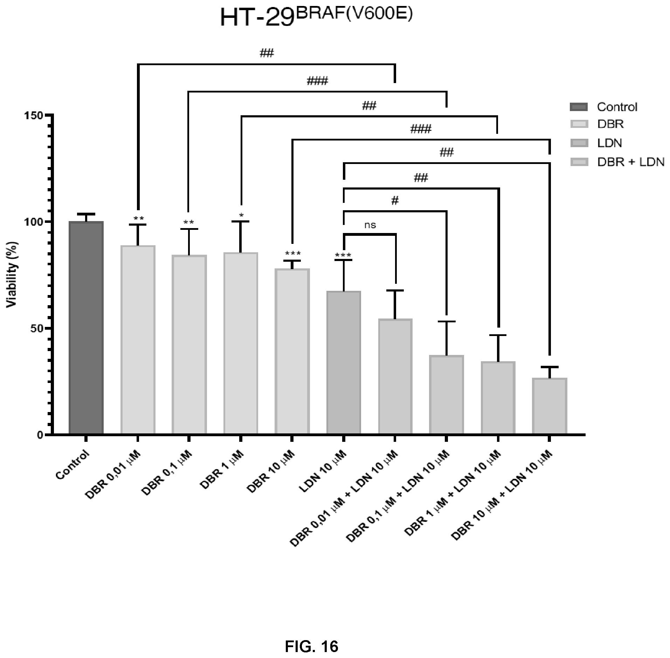

**Figure 16****. Viability of HT-29 cells in response to B-RAF inhibition, DYRK2 inhibition, or their combination.** HT-29 cells were stimulated with different doses of Dabrafenib (DBR), in the presence or absence of LDN192960 (10 µM) for 48 hours. Viability was analysed using the XTT assay. The mean ±SD of three independent experiments is shown. Statistically significant differences are indicated as *, p<0.05; **, p<0.01; ***, p<0.001 for DBR *versus* DBR + LDN; #, p<0.05; ##, p<0.01; ###, p<0.001 for LDN *versus* DBR + LDN; ns, no significant differences. DBR = Dabrafenib; LDN = LDN192960.

### DETAILED DESCRIPTION OF THE INVENTION

### Cell cultures, transfection and reagents

The HEK-293T, HeLa, CHO, and HCT116 cell lines were obtained from ATCC (American Type Culture Collection). The HT-29 cell line and CRISPR DYRK2 knock-out cell lines (MDA-MB-468 DYRK2^{+/+}, MDA-MB-468 DYRK2^{-/- ,}MDA-MB-231 DYRK2^{+/+}, MDA-MB-231 DYRK2^{-/-}, Hela DYRK2^{+/+} and Hela DYRK2^{-/-}) were also used.

The HEK-293T, HeLa, CHO, MDA-MB-468 DYRK2^{+/+}, MDA-MB-468 DYRK2^{-/-}, MDA-MB-231 DYRK2^{+/+}, MDA-MB-231 DYRK2^{-/-}, Hela DYRK2^{+/+} and Hela DYRK2^{-/-} cell lines were cultured in DMEM (Dulbecco's Modified Eagle's Medium) supplemented with 10% FBS (Fetal Bovine Serum) and 1% Penicillin/Streptomycin (SigmaAldrich). The HCT116 and HT-29 cell lines were cultured in McCoy's 5A medium (Pan-Biotech) with the same supplements. All cell lines were validated by PCR using the Geneprint10 System kit (Promega) and grown at 37 °C in a humid atmosphere with 5% CO₂. Cell lines were also routinely tested to verify the absence of Mycoplasma contamination or cross-contamination.

Transient transfections were performed using RotiFect (Carl Roth) or Lipofectamine 2000 (Invitrogen). Transfections were compensated using empty plasmid vectors. The various mutant plasmids were generated by targeted mutagenesis using the Quickchange Lightning kit (Agilent). **Table 1** shows the primers designed for targeted mutagenesis. The control and DYRK2-specific siRNAs are from Dhamarcon. The recombinant DYRK2, B-RAF and MEK1 proteins are from Merck and Fisher Scientific, respectively. λ-phosphatase and ATP are from New England Biolabs and Sigma-Aldrich. The inhibitors Dabrafenib, U0126, Analog PP1 and LDN192960 were obtained from SelleckChem, Sigma-Aldrich, Santa-Cruz Biotechnogy and MedChemExpress, respectively. The compounds XTT (2,3-Bis-(2-Methoxy-4-Nitro-5-Sulfophenyl)-2H-Tetrazolium-5-Carboxanilide) and PMS (phenazine methosulphate) are from Fisher Scientific.

**Table 1. Primers designed for mutagenesis. F: Forward; R: Reverse.**

| Initial plasmid | Primers | Final Plasmid |
|---|---|---|
| Myc MEK1 WT | **SEQ ID NO: 1:** | Myc MEK1 S385A/T386A |
| | F: 5' CCCAGGCCAAGGGCCCCCGGGAGGCCCCTTGCCTCATACGGAATG 3' | |
| | **SEQ ID NO: 2:** | |
| | R: 5' GGCTGTCCATTCCGTATGAGGCAAGGGGCCTCCCGGGGGCCCTTG 3' | |
| Myc MEK1 | **SEQ ID NO: 3:** | Myc MEK1 |
| | F: 5' CCCAGGCCAAGGGCCCCCGGGAGGCCCCTTGCCTCATACGGAATG 3' | |
| WT | **SEQ ID NO: 4:** | T292A/S298A |
| | R: 5' GGCTGTCCATTCCGTATGAGGCAAGGGGCCTCCCGGGGGCCCTTG 3' | |
| Myc MEK1 WT | **SEQ ID NO: 5:** | Myc MEK1 S218A/S222A |
| | F: 5' GGGGTCAGCGGGCAGCTCATCGACGCCATGGCCAACGCCTTCG 3' | |
| | **SEQ ID NO: 6:** | |
| | R: 5' GTAGGACCTTGTGCCCACGAAGGCGTTGGCCATGGCGTCGATG 3' | |

### mRNA extraction, reverse transcription and qPCR.

mRNA extraction was performed using the commercial High Pure RNA Isolation kit (Roche), followed by reverse transcription to cDNA using the iSCript^{™} cDNA Synthesis kit (Bio-Rad) and quantification by real-time PCR using specific primers **(Table 2),** iQTM SYBBR Green Supermix (Bio-Rad) and the iCYCLER detection System thermocycler (Bio-Rad). Finally, the fold-change, normalised with the constitutive HPRT gene, was calculated using the method2^{-ΔΔCt}. The results were expressed as mean ± SD and Student's t tests were performed, considering P<0.05 as significant. Graphs and statistical tests were performed using GraphPad Prism version 8.00 (GraphPad).

**Table 2. Primers designed for qPCR. F: Forward; R: Reverse.**

| **Protein** | | **Primers** |
|---|---|---|
| BRAF | SEQ ID NO: 7 | F: 5' AACGAGACCGATCCTCATCAGC 3' |
| | SEQ ID NO: 8 | R: 5' GGTAGCAGACAAACCTGTGGTTG 3' |
| HPRT | SEQ ID NO: 9 | F: 5' CATTATGCTGAGGATTTGGAAAGG 3' |
| | SEQ ID NO: 10 | R: 5' CTTGAGCACACAGAGGGCTACA 3' |

### Cell lysis and Western-blot.

The different cell lines were collected, washed in PBS and lysed in NP-40 buffer. The protein extracts were normalised using the Bradford technique, diluted and incubated for 5 minutes at 95 °C in 1X SDS buffer to be loaded onto SDS-PAGE gels. The proteins were then transferred to nitrocellulose membranes, which were subsequently blocked in powdered milk or 5% BSA in TBS-Tween. After blocking, the membranes were incubated with the corresponding primary antibodies overnight. Actin was used as a constitutive protein. Finally, the membranes were washed three times in TBS-Tween, incubated for one hour with the appropriate fluorescent secondary antibody, and washed three more times in TBS-Tween. The ChemiDoc MP Imaging System (Bio-Rad) was used for visualisation. The primary antibodies used were: anti-Flag (Sigma-Aldrich), anti-Myc (Roche Molecular Biochemicals), anti-DYRK2 (Sigma-Aldrich), anti-Actin (Sigma-Aldrich), anti-Actin (Abcam), anti-A-RAF (Santa Cruz), anti-B-RAF (Santa Cruz), anti-C-RAF (Santa Cruz), anti-MEK1 (Santa Cruz), anti-p-MEK1-S298 (ProteinTech), anti-p-MEK1-T292 (ProteinTech), anti-total ERK (Cell Signalling) and anti-p-ERK-T202/T204 (Cell Signalling). The fluorescent secondary antibodies used are from Bio-rad: Goat anti Mouse IgG (H/L): DyLight^{®}800, StarBright^{™} Blue 700 Goat Anti-Mouse IgG, StarBright^{™} Blue 520 Goat Anti-Mouse IgG, StarBright Blue 700 Goat Anti-Rabbit IgG, StarBright^{™} Blue 520 Goat Anti-Rabbit IgG and Rabbit F(ab')2 anti Rat IgG: Dylight^{®}800.

### Immunofluorescence.

The cells were seeded on sterile coverslips, fixed with 4% formaldehyde in PBS, permeabilised in 0.1% Triton-X100 in PBS and blocked for half an hour with a 3% BSA solution in PBS. PBS washes were performed between these steps. The blocked cells were incubated overnight with the corresponding primary antibodies diluted in 3% BSA/PBS. Finally, the cells were incubated for 1 hour with the corresponding secondary antibodies and mounted using DAPI mounting medium. The samples were washed with distilled water after antibody incubations to eliminate background noise. Fluorescence images were obtained using LSM 5 EXCITER confocal laser scanning equipment (Carl Zeiss Microlmaging GmbH) and ZEN 2008 software (Carl Zeiss Microlmaging GmbH). The RGB Profile tool of the ImageJ software (http://imagej.nih.gov/ij/) was used to analyse the fluorescence intensity of the different signals along the marked line.

### In vitro kinase assay, mass spectrometry and structural modelling.

The recombinant proteins were incubated in kinase assay buffer with or without ATP (10 µM), Dabrafenib (10 µM) and λ -phosphatase for one hour at 37°C with agitation. The reaction product was loaded in duplicate and in parallel onto two SDS-PAGE gels. One of these was used for Western-blot analysis and the other was stained with Commassie. The Commassie gel band of the protein to be analysed by MS/MS was cut out and sent to the CRG/UPF proteomics unit (Barcelona, Spain). The samples were prepared in the unit for analysis by LC-MS using LTQ-Orbitrap Fusion Lumos equipment (Thermo-Fisher Scientific) connected to EASY-nLC 1000 (Thermo-Fisher Scientific). The data obtained were analysed at the unit using the Proteome Discoverer suite version 2.3. (Thermo-Fisher Scientific) and Mascor search engine version 2.6. (Matrix Science). For the structural modelling of the protein, the complete three-dimensional structure of B-RAF and MEK1 had to be estimated using the I-Tasser programme (https://zhanglab.ccmb.med.umich.edu/I-TASSER/) and the UCSF ChimeraX molecular visualiser was used.

### Cell viability experiments.

Cell viability was analysed after exposure to various inhibitors using the XTT assay, a colorimetric assay that detects cellular metabolic activities. The cells were stimulated with different concentrations of Dabrafenib and/or LDN192960, a potent and selective DYRK2 inhibitor, for 48 hours. After stimulation, cell viability was analysed by measuring absorbance at 450 nm with TriStar-LB-941 (Berthold Technologies GmbH & Co.KG) after 4 hours of exposure to an XTT solution and PMS activation reagent in the dark. Specifically, this solution is composed of 1 mg/ml XTT and 2.5 µl/ml PMS at 0.01M.

### Results.

The new functions of DYRK2 kinase (Dual specificity tyrosine-phosphorylation-regulated kinase 2) have been investigated. This kinase had previously been linked to the control of tumorigenesis through the phosphorylation of key substrates in cancer. It is known to be involved in the regulation of key processes such as cell survival, gene transcription regulation and protein degradation, development, differentiation, among others.

Its immunoprecipitation was carried out together with the analysis of its interactors by mass spectrometry. The results were analysed in conjunction with an *in vitro* kinase assay with the aim of identifying new possible substrates of DYRK2. These approaches allowed the identification of the B-RAF kinase as a substrate of DYRK2.

To verify these results, the effect of DYRK2 on all members of the RAF family was studied. The expression of DYRK2 led to the stabilisation of all its members **(****Figure 1A****).** In view of these results, the underlying mechanism of action was sought to be determined. B-RAF was chosen for this purpose as it is the member of the RAF family with the highest basal activity and highest mutation rate in tumours. DYRK2 was re-expressed and it was observed that the stabilisation of B-RAF levels was achieved without altering its mRNA levels. These results indicate the stabilisation of B-RAF by DYRK2 through a post-transcriptional mechanism **(****Figure 1B****).** These results show that DYRK2 positively regulates B-RAF protein levels.

Next, the opposite approach was taken, and it was found that DYRK2 silencing was associated with decreased B-RAF levels **(****Figure 2A****).** Similarly, complete loss of DYRK2 via CRISPR-Cas9 in various triple-negative breast cancer lines (MDA-MB-468 and MDA-MB-231) promoted a clear decrease in endogenous B-RAF levels **(****Figure 2B****).**

It was investigated whether this effect also occurred with the most frequently mutated versions of B-RAF in tumours, specifically with the most common hyperactive mutation (B-RAF ^{V600E}) and the inactive version of B-RAF (B-RAF KM), which arises as a mechanism of resistance to inhibitors, favouring the reactivation of the pathway through its heterodimerisation with C-RAF. As can be seen in **Figure 3A****,** DYRK2 stabilises both mutant versions. This fact was also verified using a colorectal cancer cell line that presents the V600E mutation (HT-29) **(****Figure 3B****).** Taken together, these results indicate that DYRK2 stabilises B-RAF WT and its frequent mutant versions through a post-transcriptional mechanism.

To further investigate this regulatory mechanism, we studied whether DYRK2 kinase activity could be involved. To do this, we analysed the effect on B-RAF of wild-type DYRK2 (DYRK2 WT) expression compared to a mutant version of DYRK2 lacking kinase activity (DYRK2-KM). As shown in **Figure 4A****,** the stabilisation of B-RAF by DYRK2 only occurred when DYRK2 exhibited kinase activity. Similar results were obtained with reversion experiments in HeLa knock-out lines, where the absence of DYRK2 led to a drastic reduction in B-RAF that was reversed only in the presence of DYRK2 expression with kinase activity **(****Figure 4B****).**

Given that these results showed that DYRK2 kinase activity was involved, we sought to determine the ability of DYRK2 and B-RAF to interact by studying the possibility of co-localisation of both. DYRK2 and B-RAF co-localised in the cytoplasm **(****Figure 5****).**

Next, the possible phosphorylation of B-RAF by DYRK2 was analysed using an *in vitro* kinase assay. As a control for the reaction, the self-phosphorylation of DYRK2 was observed. The increase in the molecular weight of B-RAF in the presence of DYRK2 and ATP, which was reduced in the presence of λ -phosphatase, indicated a phosphorylation event **(****Figure 6A****).** As a result, this sample was sent for analysis by mass spectrometry, allowing the identification of at least three different residues (S147, S465, S729). These residues were distributed throughout the B-RAF sequence, being exposed and very close to each other in the three-dimensional structure of the protein **(****Figure 6B and 6C****).** Overall, it has been demonstrated that DYRK2 co-localises and phosphorylates B-RAF, promoting its stabilisation.

Once the role of DYRK2 on B-RAF had been determined, it was studied whether DYRK2 was regulating other members of the MAPK/ERK1/2 pathway, such as MEK1. To do this, the gradual expression of DYRK2 was carried out. As can be seen in **Figure 7A****,** this process led to a gradual increase in the molecular weight of MEK1 with a corresponding delay in electrophoretic migration. Next, we analysed whether the kinase activity of DYRK2 was involved in the delay in migration. Under this objective, it was observed that the delay in MEK1 migration occurred only in the presence of DYRK2 with kinase activity **(****Figure 7B****).**

Given that B-RAF is the upstream protein of MEK1, we wanted to rule out the possibility that the effect of DYRK2 on MEK1 was solely due to an indirect consequence of the effect of DYRK2 on B-RAF. Under this premise, different approaches were carried out. First, we analysed whether inhibition of B-RAF by its inhibitor Dabrafenib altered the increase in MEK1 molecular weight in the presence of DYRK2 **(****Figure 8A****).** Likewise, this same increase was compared between a wild-type version of MEK1 (MEK1 WT) and a mutant version of MEK1 that cannot be phosphorylated in the activation loop residues phosphorylated by B-RAF (MEK1 S218A-S222A) **(****Figure 8B****).** In both cases, it was observed that the increase in the molecular weight of MEK1 induced by the presence of DYRK2 was independent of B-RAF.

After ruling out that the effect of DYRK2 on MEK1 was due to the action of B-RAF, the possible interaction of both proteins was analysed by studying their co-localisation. As shown in **Figure 9****,** DYRK2 and MEK1 co-localised in the cytoplasm, mainly in the peri-nuclear region.

Given that both proteins co-localised and that DYRK2 kinase activity was involved, *an in vitro* kinase assay was performed to determine whether DYRK2 could also be phosphorylating MEK1. Once again, DYRK2 auto-phosphorylation was observed as a positive control for the reaction. The delay in MEK1 migration in the presence of DYRK2 and ATP alone points to a phosphorylation event in MEK1 **(****Figure 10A****).** Therefore, the samples were sent for MS/MS analysis to identify possible phosphor-sites. The analysis allowed the identification of two main pairs of possible phospho-residues: S385/T386 and T292/S298. Although these residues were distributed throughout MEK1, they were highly exposed and close to each other in three dimensions. Specifically, they were located within or near the kinase domain of MEK1 **(****Figures 10B and 10C****).**

In order to determine whether these residues were responsible for the delay in MEK1 migration promoted by DYRK2 *in vivo,* non-phosphorylatable point mutants of these residues were created. Specifically, two double mutants were created by mutating the serine or threonine residues to alanine in each pair of possible phospho-sites. After verifying their mutation by sequencing, the effect of DYRK2 on them was analysed in comparison with wild-type MEK1 (MEK1 WT). As shown in **Figure 11A****,** the double mutant MEK1 S385A/T386A responded to DYRK2 expression in the same way as MEK1 WT. In contrast, the double mutant MEK1 T292A/S298A did not experience the delay in migration observed with MEK1 WT after expressing DYRK2 **(****Figure 11B****).** This result suggested that the T292 and/or S298 residue could be responsible for the DYRK2-mediated increase in the molecular weight of MEK1. For analysis, the phosphorylation profile of these residues was studied in the presence or absence of DYRK2. In the presence of DYRK2, there was a dramatic increase in the phosphorylation of both residues *in vivo* **(****Figure 11C****).** Taken together, these results showed that DYRK2 is capable of co-localising with MEK1 and carrying out its phosphorylation at T292 and S298.

Once the underlying molecular mechanism had been characterised, the involvement of these regulations in the activation of the MAPK/ERK1/2 pathway was studied. To this end, phosphorylation in the ERK1/2 activation loop (pERK1/2-T202/T204) was monitored under various experimental circumstances. In all experiments, B-RAF stabilisation was used as a positive control to confirm that DYRK2 was acting on the MAPK/ERK1/2 pathway. First, gradient expression of wild-type DYRK2 or a mutant version of DYRK2 lacking kinase activity (DYRK2 KM) was performed. As DYRK2 expression increased, there was a greater increase in ERK1/2 activation, which only occurred if DYRK2 had kinase activity. Likewise, chemical inhibition of DYRK2 using its inhibitor LDN192960 led to a drastic reduction in the basal level of active ERK1/2 **(****Figure 12A****).**

In order to further investigate the specificity of this activation of the pathway via DYRK2, a mutant version of DYRK2 was used that was characterised by having an altered ATP binding pocket (DYRK2-GK). This alteration makes it the only protein in the cell sensitive to the analogue inhibitor PP1. As shown in **Figure 12B****,** DYRK2 causes phosphorylation of the ERK1/2 activation loop in a manner that is dependent on and completely selective for its activity.

Subsequently, the opposite approach was taken and DYRK2 expression was reduced using specific protein silencers (siRNA DYRK2). The reduction in DYRK2 protein levels or the inhibition of its activity using the inhibitor LDN192960 led to a reduction in B-RAF levels and basal activation of the pathway **(****Figure 12C****).** Taken together, these data show that the action of DYRK2 on B-RAF or MEK1 has direct consequences on the activation of the MAPK/ERK1/2 pathway. Specifically, they demonstrate that DYRK2 is a positive regulator of the pathway and promotes its basal activation.

Given that DYRK2 promoted the activation of the MAPK/ERK1/2 pathway, this activation was studied in the presence of another activator of the pathway. Specifically, epidermal growth factor (EGF). To do this, the cell line was exposed to different concentrations of this ligand in the absence or presence of DYRK2. The B-RAF inhibitor Dabrafenib was used as a negative control for pathway inactivation. As described, the MAPK/ERK1/2 pathway was gradually activated as the concentration of the EGF ligand increased. This activation was enhanced in the presence of DYRK2, indicating an additive or synergistic mechanism of DYRK2 activation with EGF. Conversely, the use of the inhibitor Dabrafenib led to a reduction in pathway activation. It should be noted that this reduction was less pronounced in the presence of DYRK2, indicating that the effect of DYRK2 not only occurs at the B-RAF level, but also promotes downstream pathway activation via MEK1. Furthermore, surprisingly, DYRK2 stabilisation was observed in the presence of the B-RAF inhibitor Dabrafenib **(****Figure 13A****).** To corroborate this observation, cells were exposed to DYRK2 expression alone or in the presence of different MAPK/ERK1/2 pathway inhibitors: a B-RAF inhibitor (Dabrafenib) and a MEK1 inhibitor (U0126). After 24 hours of exposure to these inhibitors, the cells responded by increasing DYRK2 levels **(****Figure 13B****).** This result shows a new and hitherto unknown mechanism of resistance to MAPK/ERK pathway inhibitors. Specifically, the cells respond after prolonged inactivation of the pathway by increasing its positive regulator, DYRK2.

### Treatment with a DYRK2 inhibitor (LDN192960) in combination with a clinical inhibitor of the pathway (Dabrafenib) in tumours with hyperactive MAPK/ERK1/2 pathway.

Various tumour cell lines representative of tumours where it is frequently dysregulated and its various associated mutations or alterations are used.

### Tumours with mutated RAS causing inactivation of the pathway.

This type of mutation is mainly common in colon cancer tumours (45%) and non-small cell lung cancer (35%). To analyse the effect of DYRK2 inhibitors, colorectal cancer (HCT116) and lung cancer (A549) cell lines with mutated RAS were exposed to different doses of the DYRK2 inhibitor LDN192960 alone or in combination with the B-RAF inhibitor Dabrafenib.

As previously mentioned, the use of B-RAF inhibitors has been restricted to tumours that only present mutated B-RAF. This is because upstream mutations, such as RAS mutations, can increase pathway activation at low doses through paradoxical effects. Thus, for tumours with mutated RAS, the use of B-RAF or MEK1/2 inhibitors, which are currently the most specific inhibitors of the pathway, is disabled.

As shown in **Figure 14** and **Figure 15****,** colon and lung cancer cells with the RAS mutation showed an increase in tumour survival compared to the control at low doses of the B-RAF inhibitor Dabrafenib. This result shows both cell lines to be a good model for studying tumour behaviour. Only at very high doses is this inhibitor capable of slightly reducing cell viability. However, the DYRK2 inhibitor LDN192960 had a more potent effect on reducing cell viability in these lines compared to Dabrafenib. This anti-tumour effect is enhanced in the presence of both inhibitors in a dose-dependent manner. These results show DYRK2 to be a better target for the treatment of tumours with mutated RAS. It also corroborates the hypothesis that DYRK2 inhibition would avoid paradoxical effects by decreasing the ability to form heterodimers. On the other hand, it should be noted that in the case of HCT116 cells, the PI3K pathway is also hyperactive, which is one of the frequent mechanisms in the recurrence of these tumours. However, inhibition of DYRK2 causes a reduction in tumour survival, pointing to the protein as a major therapeutic target.

### Tumours with mutated B-RAF.

Of the three RAF isoforms, B-RAF has the highest mutation rate (8%) in human cancers. To date, more than 200 mutations have been described in human cancers, of which the B-RAF^{V600E} mutation accounts for 95% of all B-RAF mutations. This B-RAF^{V600E} mutation is included within the class I mutations of B-RAF, which are characterised by causing a much higher kinase activity and allowing B-RAF to act as a monomer independently of RAS. Due to its high frequency in human cancers, this mutation has been the target of most inhibitors developed against B-RAF.

To evaluate the effect of DYRK2 inhibitors alone or in combination with specific inhibitors of pathway components on tumours with mutated B-RAF, the HT-29 colorectal cancer cell line, which has the B-RAF^{V600E} mutation, was used. As shown in **Figure 16****,** HT-29 cells experienced a dose-dependent decrease in viability in response to the B-RAF inhibitor Dabrafenib. This decrease in cell viability was greater in the presence of the DYRK2 inhibitor LDN192960 and even greater when both inhibitors were combined. These results demonstrate that DYRK2 inhibition in combination with B-RAF inhibition may be a more effective therapeutic strategy in the treatment of tumours with mutated B-RAF.

### References.

1. Guo, Y.-J. et al. ERK/MAPK signalling pathway and tumorigenesis. Exp. Ther. Med. 19, 1997 (2020).
2. Ullah, R., Yin, Q., Snell, A. H. & Wan, L. RAF-MEK-ERK pathway in cancer evolution and treatment. Semin. Cancer Biol. 85, 123-154 (2022).
3. Moon, H. & Ro, S. W. MAPK/ERK Signalling Pathway in Hepatocellular Carcinoma. Cancers (Basel). 13, (2021).
4. Yuan, J., Dong, X., Yap, J. & Hu, J. The MAPK and AMPK signalings: interplay and implication in targeted cancer therapy. J. Haematol. Oncol. 13, (2020).
5. Morrison, D. K. MAP kinase pathways. Cold Spring Harb. Perspect. Biol. 4, (2012).
6. Śmiech, M., Leszczyński, P., Kono, H., Wardell, C. & Taniguchi, H. Emerging BRAF Mutations in Cancer Progression and Their Possible Effects on Transcriptional Networks. Genes (Basel). 11, 1-14 (2020).
7. Song, Y. et al. Targeting RAS-RAF-MEK-ERK signalling pathway in human cancer: Current status in clinical trials. Genes Dis. 10, (2022).
8. Rocca, A., Braga, L., Volpe, M. C., Maiocchi, S. & Generali, D. The Predictive and Prognostic Role of RAS-RAF-MEK-ERK Pathway Alterations in Breast Cancer: Revision of the Literature and Comparison with the Analysis of Cancer Genomic Datasets. Cancers (Basel). 14, (2022).
9. Furukawa, T. Impacts of activation of the mitogen-activated protein kinase pathway in pancreatic cancer. Front. Oncol. 5, (2015).
10. Kwan, A. K., Piazza, G. A., Keeton, A. B. & Leite, C. A. The path to the clinic: a comprehensive review on direct KRASG12C inhibitors. J. Exp. Clin. Cancer Res. 41, (2022).
11. Ni, D. et al. Drugging K-RasG12C through covalent inhibitors: Mission possible? Pharmacol. Ther. 202, 1-17 (2019).
12. Gouda, M. A. & Subbiah, V. Precision oncology for BRAF-mutant cancers with BRAF and MEK inhibitors: from melanoma to tissue-agnostic therapy. ESMO open 8, (2023).
13. Cox, A. D. & Der, C. J. The raf inhibitor paradox: unexpected consequences of targeted drugs. Cancer Cell 17, 221-223 (2010).
14. Zhang, C. et al. RAF inhibitors that evade paradoxical MAPK pathway activation. Nature 526, 583-586 (2015).
15. Yoshida, S. & Yoshida, K. Multiple functions of DYRK2 in cancer and tissue development. FEBS Lett. 593, 2953-2965 (2019).
16. Correa-Sáez, A. et al. Updating dual-specificity tyrosine-phosphorylation-regulated kinase 2 (DYRK2): molecular basis, functions and role in diseases. Cell. Mol. Life Sci. 77, 4747-4763 (2020).
17. Tandon, V., De La Vega, L. & Banerjee, S. Emerging roles of DYRK2 in cancer. J. Biol. Chem. 296, (2021).
18. Sun, Q. & Wang, W. Structures of BRAF-MEK1-14-3-3 sheds light on drug discovery. Signal Transduct. Target. Ther. 4, (2019).
19. Haling, J. R. et al. Structure of the BRAF-MEK complex reveals a kinase activity independent role for BRAF in MAPK signalling. Cancer Cell 26, 402-413 (2014).
20. Gnad, F. et al. Phosphoproteome analysis of the MAPK pathway reveals previously undetected feedback mechanisms. Proteomics 16, 1998-2004 (2016).
21. Cope, N. et al. Biochemical Characterisation of Full-Length Oncogenic BRAFV600E together with Molecular Dynamics Simulations Provide Insight into the Activation and Inhibition Mechanisms of RAF Kinases. Chembiochem 20, 2850-2861 (2019).
22. Cope, N. et al. Mechanism of BRAF Activation through Biochemical Characterisation of the Recombinant Full-Length Protein. Chembiochem 19, 1988-1997 (2018).
23. Eblen, S. T. et al. Mitogen-activated protein kinase feedback phosphorylation regulates MEK1 complex formation and activation during cellular adhesion. Mol. Cell. Biol. 24, 2308-2317 (2004).
24. Skarpen, E. et al. MEK1 and MEK2 regulate distinct functions by sorting ERK2 to different intracellular compartments. FASEB J. 22, 466-476 (2008).
25. Wei, T. et al. Selective inhibition reveals the regulatory function of DYRK2 in protein synthesis and calcium entry. Elife 11, (2022).
26. Yuan, K. et al. Discovery of Potent DYRK2 Inhibitors with High Selectivity, Great Solubility, and Excellent Safety Properties for the Treatment of Prostate Cancer. J. Med. Chem. 66, 4215-4230 (2023).

## Claims

1. A DYRK2 inhibitor for use as an inhibitor of the MAPK/ERK1/2 pathway.

2. The DYRK2 inhibitor for use according to the preceding claim, wherein the inhibitor is selected from the list comprising YK-2-69, C17, C43, GSK626616, Leucettine L41, EHT 5372, EHT 1610, TG003, INDY, DYR219, SM07883, RD0392, Abbott COT/TPL2 compound 41, Fluoro-DANDY analogue 5 g, SC97202, LY2835219, Milciclib, PAC1, Compound 6i, Curcumin, Harmine, Ro3306, BX-795, A-443654, AnnH75, WNK-463, GNF2133, LDN192960 and their derivatives.

3. The DYRK2 inhibitor according to claims 1 or 2 for use as a medicament in the treatment of cancers **characterised by** the hyperactivation of the MAPK/ERK1/2 pathway.

4. The DYRK2 inhibitor for use according to the previous claim, wherein the type of cancer is selected from the list comprising: pancreatic cancer, colon cancer, bladder cancer, non-small cell lung cancer, acute lymphocytic leukaemia, acute myeloid leukaemia, melanoma, thyroid cancer, ovarian cancer, hepatocellular carcinoma, breast cancer, and pancreatic cancer.

5. A pharmaceutical composition comprising at least one DYRK2 inhibitor for use in the treatment of cancers **characterised by** the hyperactivation of the MAPK/ERK1/2 pathway.

6. The pharmaceutical composition according to the previous claim, which also comprises at least one inhibitor of a receptor of the MAPK/ERK1/2 pathway other than the DYRK2 inhibitor.

7. The pharmaceutical composition according to the previous claim, **characterised in that** the inhibitor of a receptor of the MAPK/ERK1/2 pathway is a B-RAF inhibitor.

8. The pharmaceutical composition according to the preceding claim, **characterised in that** the B-RAF inhibitor is selected from the list comprising Vemurafenib, Dabrafenib, Encorafenib, PLX-4720, RAF-265, GDC-0879, AZ-628, SB590885, ZM336372, GW5074, TAK-632, Donafenib, Agerafenib, Belvarafenib, GNE-9815 and their derivatives.

9. The pharmaceutical composition according to claim 6, **characterised in that** the inhibitor of a receptor of the MAPK/ERK1/2 pathway is a MEK1/2 inhibitor.

10. The pharmaceutical composition according to the previous claim **characterised in that** the MEK1/2 inhibitor is selected from the list comprising Trametinib, Binimetinib, Cobimetinib, U0126, Avutometinib, Selumetinib, Mirdametinib, PD98059, PD184352, Pimasertib, BIX 02189, Pelitinib, TAK-733, AZD8330, Refametinib, Zapnometinib, GDC-0623 and BI-847325 and their derivatives.

11. The pharmaceutical composition according to claim 6, **characterised in that** it comprises at least one B-RAF inhibitor and one MEK1/2 inhibitor.

12. The pharmaceutical composition according to the previous claim, **characterised in that** the B-RAF inhibitor is Dabrafenib and the MEK1/2 inhibitor is U0126.

13. The pharmaceutical composition according to claim 6, **characterised in that** the inhibitor of a receptor of the MAPK/ERK1/2 pathway is a RAS inhibitor.

14. The pharmaceutical composition according to the preceding claim, **characterised in that** the RAS inhibitor is selected from the list comprising Sotorasib, Adagrasib, MCP110, Lonafarnib, JDQ443, Pan-RAS-IN-1, ASP2453, BI-3406, BI-2852, Kobe2602, BAY-293, KRpep-2d, ARS-853, ARS-1620, TH-Z816 and their derivatives.

15. The pharmaceutical composition according to claim 6, **characterised in that** the the inhibitor of a receptor of the MAPK/ERK1/2 pathway is an ERK1/2 inhibitor.

16. The pharmaceutical composition according to the previous claim **characterised in that** the ERK1/2 inhibitor is selected from the list comprising Imipridone, SCH772984, ASN007, MRTX-1257. AZD0364, MK-8353, Temuterkib, Pluripotin, VX-11e, Ulixertinib, Ravoxertinib, FR 180204 and their derivatives.

17. The pharmaceutical composition according to any of claims 6 to 16, **characterised in that** it further comprises inhibitors of positive regulators of the MAPK/ERK1/2 pathway such as COT, RAS-GEF, AMPK, PKA, PAK1 or CAMPK and/or stabilisers of negative regulators of the MAPK/ERK1/2 pathway such as DUSP6, DUSP1, NF1, RAS-GAP, KSR, Sprouty or RKIP.
